(19) Europäisches Patentamt
European Patent Office
Office européen des brevets

(11) Numéro de publication : **0 153 230**
**B1**

# (12) FASCICULE DE BREVET EUROPÉEN

(45) Date de publication du fascicule du brevet : **19.07.89**

(21) Numéro de dépôt : **85400219.3**

(22) Date de dépôt : **11.02.85**

(51) Int. Cl.[4] : **C 07 D513/04**, C 07 D513/10, A 61 K 31/505, C 07 D277/82 // (C07D513/04, 239:00, 277:00)

(54) **Nouveaux dérivés du pyrimido /2,1-b/ benzothiazole et leurs sels, procédé et intermédiaires de préparation, application à titre de médicaments et compositions les renfermant.**

(30) Priorité : **13.02.84 GB 8403739**

(43) Date de publication de la demande : **28.08.85 Bulletin 85/35**

(45) Mention de la délivrance du brevet : **19.07.89 Bulletin 89/29**

(84) Etats contractants désignés : **AT BE CH DE FR IT LI LU NL SE**

(56) Documents cités :
**EP-A- 0 005 154**
**US-A- 3 919 201**
**CHEMICAL ABSTRACTS, vol. 91, 1979, page 638, ref. no. 193220g, Columbus, Ohio, US. KANG-CHIEN LIU et al.: "Synthesis and bioactivities of some 2-oxo-naphtho(1,2-d)thiazolo(3,2-a)pyrimidine derivatives"**

(73) Titulaire : **ROUSSEL-UCLAF**
**35, boulevard des Invalides**
**F-75007 Paris (FR)**

(72) Inventeur : **Rowlands, David Alun**
**Wyke House Crudwell**
**Malmesbury Wiltshire (GB)**
Inventeur : **Golec, Julian Marian Charles**
**23A Dixon Street**
**Swindon Wiltshire (GB)**

(74) Mandataire : **Vieillefosse, Jean-Claude et al**
**Département des Brevets ROUSSEL UCLAF B.P no 9**
**F-93230 Romainville (FR)**

EP 0 153 230 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet de nouveaux dérivés du pyrimido [2,1-b] benzothiazole, ainsi que leurs sels, le procédé et des intermédiaires de préparation et l'application à titre de médicament de ces nouveaux produits.

Le brevet américain US-A-3919201 décrit des produits présentant à la fois des substituants différents sur le noyau tricyclique en position 8 et des propriétés pharmacologiques différentes (antifungiques, anti-amibiens et anti-inflammatoires).

L'invention a pour objet des dérivés du pyrimido [2,1-b] benzothiazole ainsi que leurs sels, caractérisés en ce qu'ils répondent à la formule générale (I) :

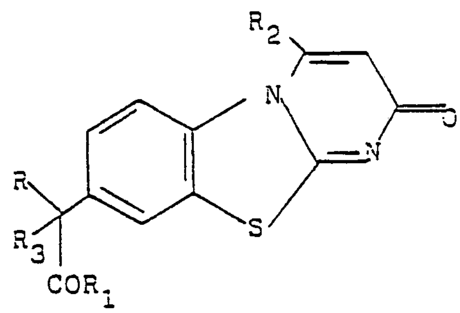

(I)

dans laquelle R et $R_3$, identiques ou différents, représentent un atome d'hydrogène, un radical alcoyle, linéaire ou ramifié, renfermant de 1 à 6 atomes de carbone ou R et $R_3$ forment avec l'atome de carbone auquel ils sont liés, un radical cycloalcoyle renfermant de 3 à 6 atomes de carbone, $R_1$ représente un radical hydroxy, un radical alcoxy, linéaire ou ramifié, renfermant de 1 à 6 atomes de carbone ou un radical :

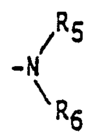

dans lequel $R_5$ et $R_6$, identiques ou différents, représentent un atome d'hydrogène, un radical alcoyle renfermant de 1 à 6 atomes de carbone ou $R_5$ et $R_6$ forment avec l'atome d'azote auquel ils sont liés un radical pipéridino ou morpholino, $R_2$ représente urt atome d'hydrogène, un radical alcoyle, linéaire ou ramifié, renfermant de 1 à 6 atomes de carbone, un radical aryle renfermant de 6 à 12 atomes de carbone, un radical aryle renfermant de 6 à 12 atomes de carbone substitué par un ou plusieurs radicaux alcoyles ou alcoxy renfermant de 1 à 6 atomes de carbone, par un ou plusieurs atomes d'halogène ou par un ou plusieurs radicaux nitro ou $R_2$ représente un radical 2-thiényle, un radical aralcoyle renfermant de 7 à 13 atomes de carbone, un radical alcoxycarbonyle renfermant de 2 à 7 atomes de carbone ou un radical cycloalcoyle renfermant de 3 à 6 atomes de carbone.

Dans la formule générale (I) et dans ce qui suit, le terme radical alcoyle renfermant de 1 à 6 atomes de carbone désigne par exemple un radical méthyle, éthyle, propyle, isopropyle, butyle, isobutyle, pentyle ou hexyle ; le terme radical alcoxy renfermant de 1 à 6 atomes de carbone désigne, par exemole, un radical méthoxy, éthoxy, propoxy, isopropoxy, butoxy, pentoxy ou hexyloxy ; le terme radical cycloalcoyle renfermant de 3 à 6 atomes decarbone désigne de préférence le radical cyclohexyle ; le terme radical aralcoyle renfermant de 7 à 13 atomes de carbone désigne de préférence un radical benzyle ; le terme radical aryle renfermant de 6 à 12 atomes de carbone désigne par exemple un radical phényle ou naphtyle ; le terme radical alcoxycarbonyle renfermant de 2 à 7 atomes de carbone désigne par exemple un radical méthoxy, éthoxy ou propoxy-carbonyle ; le terme atome d'halogène désigne par exemple un atome de fluor, de chlore ou de brome.

Les produits de formule (I) dans laquelle $R_1$ représente un radical hydroxy présentent un caractère acide ; ils peuvent former des sels métalliques ou des sels d'addition avec les bases azotées.

Les sels métalliques peuvent être par exemple, les sels de métaux alcalins, tels le sodium, le potassium ou le lithium, de métaux alcalino-terreux, tels que le calcium ou de métaux tels l'aluminium ou le magnésium.

Les sels de bases azotées peuvent être par exemple, les sels d'ammonium ou les sels d'amines, telles la trométhamine, la lysine, l'arginine, la triéthanolamine.

Les produits de formule (I) dans laquelle $R_1$ représente un radical alcoxy ou un radical $—NR_5R_6$ présentent un caractère basique et peuvent par conséquent former des sels d'addition avec les acides tels que, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcane sulfoniques, tels que l'acide méthane sulfonique et aryl sulfoniques, tels que l'acide benzène sulfonique.

Parmi les produits objet de l'invention, on peut citer notamment les dérivés répondant à la formule (I) ci-dessus, ainsi que leurs sels, caractérisés en ce que dans ladite formule (I), R et $R_3$ représentent un atome d'hydrogène ou un radical méthyle ou éthyle, $R_1$ et $R_2$ ont la signification déjà indiquée.

Parmi les produits objet de l'invention, on retient plus particulièrement les dérivés répondant à la formule (I) ci-dessus, ainsi que leurs sels, caractérisés en ce que dans ladite formule (I), R et $R_3$ représentent un atome d'hydrogène ou un radical méthyle ou éthyle, $R_1$ a la signification déjà indiquée, $R_2$ représente un atome d'hydrogène, un radical alcoyle renfermant de 1 à 6 atomes de carbone, un radical phényle, un radical phényle substitué par un ou plusieurs radicaux méthyle par un ou plusieurs atomes d'halogène ou par un ou plusieurs radicaux nitro ou méthoxy ou $R_2$ représente un radical méthoxy carbonyle.

Parmi ces derniers, on peut citer tout particulièrement les dérivés du pyrimido [2,1-b] benzothiazole dont les noms suivent :

— le 2-oxo-4-phényl-2H-pyrimido [2,1-b] benzothiazole-8-acétate d'éthyle ;
— le 2-oxo-4-(p-méthoxy phényl)-2H-pyrimido [2,1-b] benzothiazole-8-acétate d'éthyle ;
— le 2-oxo-4-(o-chlorophényl)2H-pyrimido [2,1-b] benzothiazole-8-acétate d'éthyle ;
— l'α-méthyl-2-oxo-4-phényl-2H-pyrimido [2,1-b] benzothiazole-8-acétate d'éthyle ;
— l'α-méthyl-2-oxo-4-(p-chlorophényl)-2H-pyrimido [2,1-b] benzothiazole-8-acétate d'éthyle ;
— l'α-éthyl-2-oxo-4-phényl-2H-pyrimido [2,1-b] benzothiazole-8-acétate d'éthyle ;

ainsi que leurs sels d'addition avec les acides.

L'invention a également pour objet un procédé de préparation des nouveaux dérivés du pyrimido [2,1-b] benzothiazole tels que définis ci-dessus, ainsi que de leurs sels, caractérisé en ce que l'on fait réagir un produit de formule (II) :

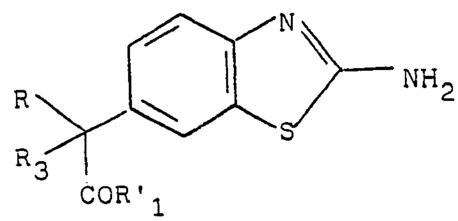

(II)

dans laquelle R et $R_3$ ont la signification déjà indiquée, $R'_1$ représente un radical hydroxy ou un radical alcoxy renfermant de 1 à 6 atomes de carbone avec un produit de formule (III) :

$$R_2—C≡C—CO_2R_4 \quad \text{(III)}$$

dans laquelle $R_2$ a la signification déjà indiquée et $R_4$ représente un radical alcoyle renfermant de 1 à 3 atomes de carbone, pour obtenir un produit de formule ($I_A$) :

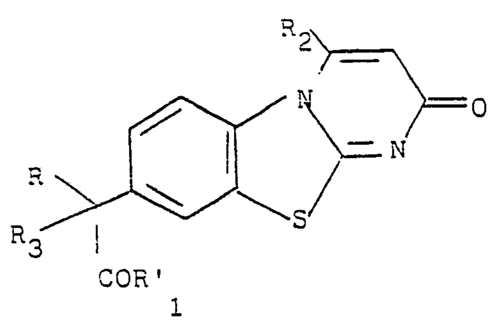

(IA)

dans laquelle R, $R_3$, $R'_1$ et $R_2$ ont la signification déjà indiquée et que :

— soit on isole et si désiré, salifie le produit de formule (I) ainsi obtenu ;

— soit on saponifie un produit de formule ($I_A$) ainsi obtenu dans laquelle $R'_1$ représente un radical alcoxy, pour obtenir un produit de formule ($I_B$) :

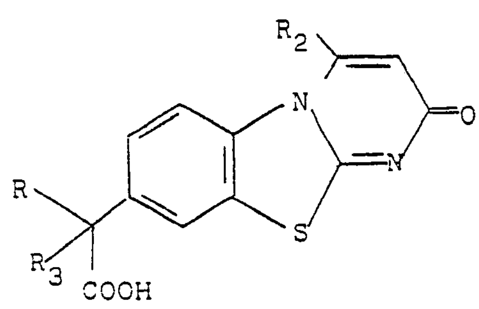

(IB)

dans laquelle R, $R_3$ et $R_2$ ont la signification déjà indiquée que, le cas échéant, l'on isole et, si désiré, salifie ou que l'on estérifie par réaction avec un alcool de formule (IV) :

$$HO—R''_1 \qquad (IV)$$

dans laquelle $R''_1$ représente un radical alcoyle renfermant de 1 à 6 atomes de carbone, pour obtenir un produit de formule (I) correspondant dans laquelle $R_1$ a la signification de $R''_1$ et, si désiré, salifie ce dernier ;

— soit on transestérifie un produit de formule ($I_A$) dans laquelle $R'_1$ représente un radical alcoxy, par action d'un alcool de formule (IV) :

$$HO—R''_1 \qquad (IV)$$

dans laquelle $R''_1$ a la signification déjà indiquée, pour obtenir un produit de formule (I) correspondant et, si désiré, salifie ce dernier ;

— soit on fait réagir un produit de formule ($I_A$) dans laquelle $R'_1$ représente un radical hydroxy, avec un produit de formule :

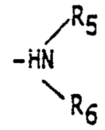

dans laquelle $R_5$ et $R_6$ ont la signification déjà indiquée, pour obtenir un produit de formule (I) correspondant et, si désiré, salifie ce dernier.

Dans des conditions préférentielles de mise en œuvre de l'invention, le procédé de préparation ci-dessus décrit est caractérisé en ce que :

a) la réaction du produit de formule (II) avec le produit de formule (III) est effectuée dans le cas où $R_2$ représente un atome d'hydrogène, au reflux du mélange réactionnel, au sein d'un alcanol tel que l'éthanol et en présence d'un catalyseur tel que le palladium ;

b) la réaction du produit de formule (II) avec le produit de formule (III) est effectuée dans le cas où $R_2$ ne représente pas un atome d'hydrogène, à une température de 100 à 180 °C, en l'absence de solvant ;

c) la saponification du produit de formule ($I_A$) est effectuée au moyen d'une base faible telle que le carbonate de potassium ;

d) la réaction du produit de formule ($I_A$) avec le produit de formule $HNR_5R_6$ est effectuée en présence de 1,1'-carbonyldiimidazole.

Les sels peuvent être préparés en faisant réagir en proportions sensiblement stœchiométriques, le cas échéant, soit une base notamment une base alcaline, alcalino-terreuse ou une base azotée, soit un acide minéral ou organique avec un produit de formule (I).

Les produits objet de la présente invention possèdent de très intéressantes propriétés pharmacologiques ; ils sont doués notamment de remarquables propriétés antiallergiques.

Ces propriétés sont illustrées plus loin dans la partie expérimentale.

Ces propriétés justifient l'utilisation des nouveaux dérivés du pyrimido [2,1-b] benzothiazole, objet de la présente invention, ainsi que de leurs sels pharmaceutiquement acceptables, à titre de médicaments.

La présente demande a ainsi également pour objet l'application à titre de médicaments des nouveaux dérivés du pyrimido [2,1-b] benzothiazole tels que définis par la formule (I), ainsi que de leurs sels d'addition avec les bases ou les acides pharmaceutiquement acceptables.

Parmi les médicaments objet de l'invention, on retient de préférence les médicaments caractérisés en ce qu'ils sont constitués par les dérivés répondant à la formule (I) dans laquelle R et $R_3$ représentent un atome d'hydrogène ou un radical méthyle ou éthyle, $R_1$ et $R_2$ ont la signification déjà indiquée, ainsi que par leurs sels d'addition avec les bases ou les acides pharmaceutiquement acceptables.

Parmi ceux-ci, on retient notamment les médicaments caractérisés en ce qu'ils sont constitués par les dérivés répondant à la formule (I) dans laquelle R et $R_3$ représentent un atome d'hydrogène ou un radical méthyle ou éthyle, $R_1$ a la signification déjà indiquée, $R_2$ représente un atome d'hydrogène, un radical alcoyle renfermant de 1 à 6 atomes de carbone, un radical phényle, un radical phényle substitué par un ou plusieurs radicaux méthyle, par un ou plusieurs atomes d'halogène, ou par un ou plusieurs radicaux nitro ou méthoxy ou $R_2$ représente un radical méthoxy carbonyle, ainsi que par leurs sels d'addition avec les bases ou les acides pharmaceutiquement acceptables.

Parmi ces derniers, on retient tout particulièrement les produits dont les noms suivent :

— le 2-oxo-4-phényl-2H-pyrimido [2,1-b] benzothiazole-8-acétate d'éthyle,
— le 2-oxo-4-(p-méthoxy phényl)-2H-pyrimido [2,1-b] benzothiazole-8-acétate d'éthyle,
— le 2-oxo-4-(o-chlorophényl)-2H-pyrimido [2,1-b] benzothiazole-8-acétate d'éthyle,
— l'α-méthyl-2-oxo-4-phényl-2H-pyrimido [2,1-b] benzothiazole-8-acétate d'éthyle,
— l'α-méthyl-2-oxo-4-(p-chlorophényl)-2H-pyrimido [2,1-b] benzothiazole-8-acétate d'éthyle,

— l'α-éthyl-2-oxo-4-phényl-2H-pyrimido [2,1-b] benzothiazole-8-acétate d'éthyle, ainsi que leurs sels d'addition avec les bases ou les acides pharmaceutiquement acceptables.

Ces médicaments trouvent, par exemple, leur emploi dans le traitement de l'asthme allergique et des bronchites asthmatiformes d'origine allergique.

La dose usuelle, variable selon le produit utilisé, le sujet traité et l'affection en cause peut être, par exemple, de 1 mg à 1000 mg par jour, par voie orale chez l'homme.

L'invention a également pour objet les compositions pharmaceutiques qui renferment au moins un dérivé précité ou l'un de ses sels d'addition avec les bases ou les acides pharmaceutiquement acceptables, à titre de principe actif.

A titre de médicaments, les dérivés répondant à la formule (I) et leurs sels d'addition avec les bases ou les acides pharmaceutiquement acceptables peuvent être incorporés dans des compositions pharmaceutiques destinées à la voie digestive, parentérale ou locale.

Ces compositions pharmaceutiques peuvent être par exemple, solides ou liquides et se présenter sous les formes pharmaceutiques couramment utilisées en médecine humaine, comme, par exemple, les comprimés, simples ou dragéifiés, les gélules, les granulés, les suppositoires, les sirops, les aérosols, les crèmes, les pommades, les préparations injectables, elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Certains produits de formule (II) sont décrits dans la littérature et notamment par S. N. Sawhney et al. dans Ind. J. Chem. 16 B, 605 (1978), ainsi que dans la demande allemande 2.015.158, dans le brevet US 3.656.958 et dans la demande européenne 17543 A.

Toutefois, certains composés de formule (II) sont nouveaux et l'invention a ainsi pour objet les produits de formule (II) :

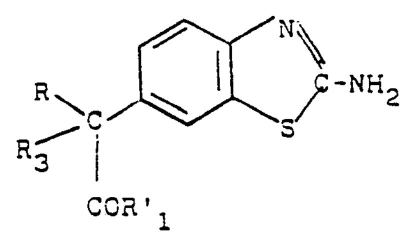

(II)

dans laquelle R, $R_3$ et $R'_1$ ont la signification précitée, étant entendu que lorsque $R'_1$ représente un radical hydroxy, le groupement $R_3RC<$ est différent de $H_3CC<H$ ou de $H_2C<$ et que lorsque $R'_1$ représente un radical éthoxy, le groupement $R_3RC<$ est différent de $H_2C<$.

Les produits de formule (II) dans laquelle R et $R_3$ représentent un atome d'hydrogène lorsqu'ils ne sont pas connus, peuvent être préparés selon le schéma suivant :

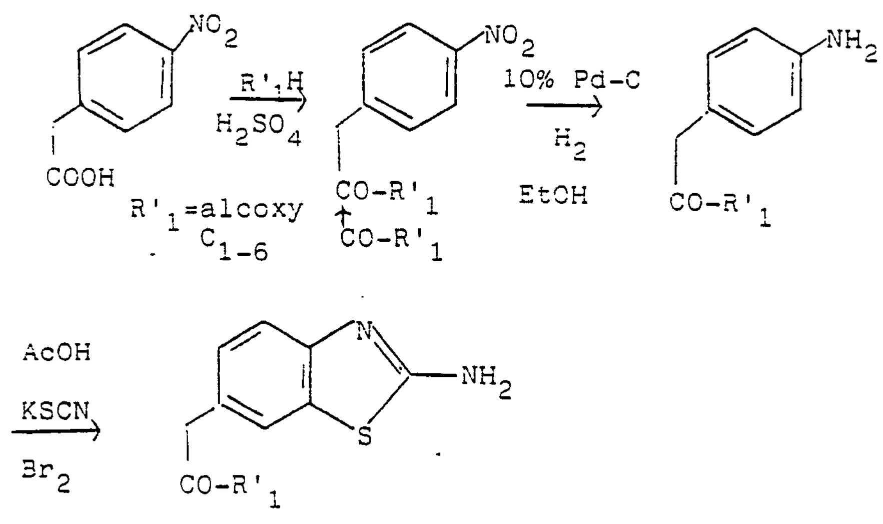


Les produits de formule (II) peuvent être également préparés au départ d'α-phényl aldéhydes selon le schéma suivant :

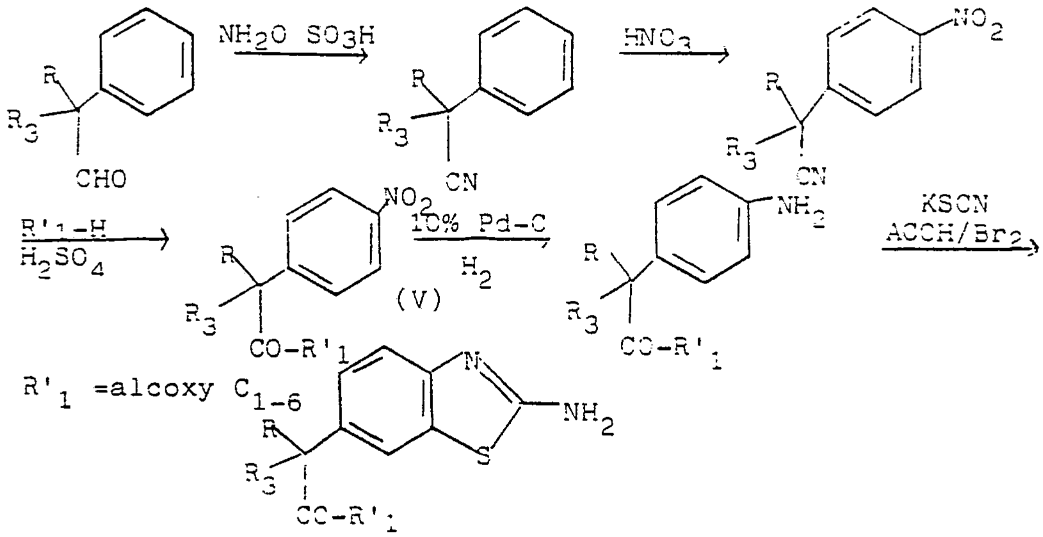


Lorsque l'on veut préparer selon le procédé ci-dessus des produits dans lesquels R et $R_3$ représentent un radical alcoyle, il est préférable de préparer l'intermédiaire de formule (V) :

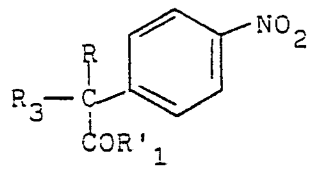

(V)

par alcoylation d'un produit de formule (VI) :

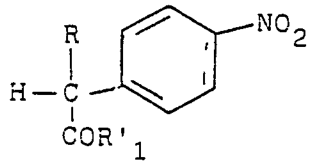

(VI)

dans laquelle R représente un radical alcoyle et $R'_1$ un radical alcoxy, en utilisant, par exemple, un iodure d'alcoyle $R_3I$ et une base telle que le diisopropylamidure de lithium ou le N-isopropylcyclohexyl amidure de lithium, au sein d'un solvant tel que le tétrahydrofuranne.

La préparation des produits de formule (VI) est décrite plus loin.

Les produits de formule (II) dans laquelle $R_3R{<}C$ représente un radical $CH_3C{<}H$ peuvent, selon une variante, également être préparés selon le schéma suivant :

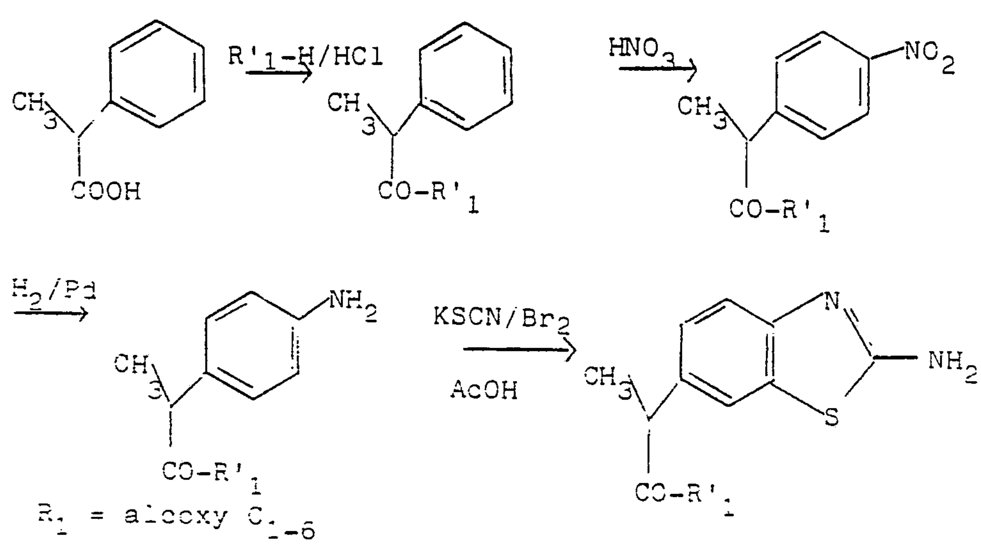

Les produits de formule (II) dans laquelle $R_3$ (ou R) représente un atome d'hydrogène, peuvent aussi être préparés au départ de 1-chloro 4-nitrobenzène selon le schéma suivant :

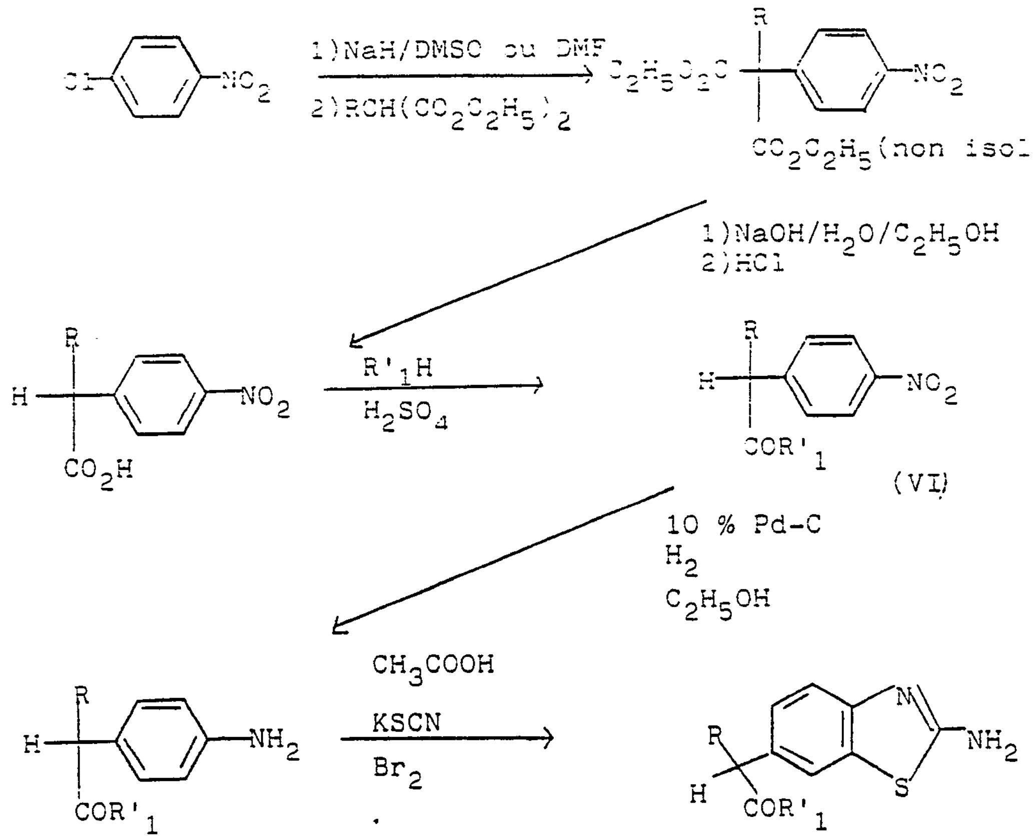


Dans le schéma qui précède R est un atome d'hydrogène ou un radical alcoyle $C_{1-6}$ de préférence méthyle ou éthyle et $R'_1$ est un radical alcoxy $C_{1-6}$.

Les premiers stades sont inspirés du procédé décrit par K. Hino et al. J. Med. Chem. 26 222-226 (1983).

Il va être donné maintenant, à titre non limitatif, des exemples de mise en œuvre de l'invention.

Exemple 1 : 2-oxo-2H-pyrimido[2,1b]benzothiazole 8-acétate d'éthyle.

On chauffe au reflux pendant 2 heures, un mélange comprenant 5 g de 2-amino benzothiazole-6-acétate d'éthyle préparé selon SAWHNEY Ind. J. Chem. 16B 605 (1978) et 1,95 g de propiolate de méthyle dans 30 $cm^3$ d'éthanol. On laisse refroidir, filtre le produit brut, le recristallise dans l'alcool n-butylique et obtient 2,88 g de produit attendu.

Exemple 2 : α-méthyl-2-oxo-2H-pyrimido [2,1-b] benzothiazole-8-acétate d'éthyle.

En utilisant une méthode analogue à celle décrite à l'exemple 1 mais en partant de l'α-méthyl 2-amino benzothiazole-6-acétate d'éthyle et en maintenant le reflux pendant 17 heures. On obtient le produit attendu avec un rendement de 42 %.

L'α-méthyl 2-amino benzothiazole 6-acétate d'éthyle utilisé au départ de l'exemple 2 a été préparé selon l'une des 2 méthodes suivantes.

Préparation A.

Stade a : α-méthyl 2-amino benzothiazole 6-acétonitrile.

On mélange sous agitation à température ambiante 25 g de 2-(p-aminophényl) propionitrile (préparé selon le brevet anglais BP 1.198.212), 66,8 g de thiocyanate de potassium dans 300 $cm^3$ d'acide acétique, ajoute lentement une solution de 27,4 g de brome dans 87,5 $cm^3$ d'acide acétique et chauffe 2 heures à 50 °C, puis verse le mélange réactionnel dans 1,5 litre d'eau. On filtre, précipite le produit brut par addition de bicarbonate de soude, filtre le précipité, le recristallise dans un mélange eau-méthanol et récupère 19,4 g de produit attendu.

F = 165,1 °C

Analyse : $C_{10}H_9N_3S$

| | | | | |
|---|---|---|---|---|
| Calculé : | C 59,09 | H 4,46 | N 20,67 | S 15,77 % |
| Trouvé : | C 58,76 | H 4,42 | N 20,74 | S 15,74 % |

Stade b : α-méthyl 2-amino benzothiazole 6-acétate d'éthyle.

On chauffe au reflux pendant 20 heures, 0,5 g de produit obtenu au stade a dans une solution contenant 10 g d'acide sulfurique concentré dans 10 $cm^3$ d'éthanol. On refroidit, dilue avec de l'eau et évapore le solvant. On neutralise la solution à l'aide de bicarbonate de sodium, filtre le précipité et le recristallise dans un mélange eau-méthanol et obtient 0,38 g de produit attendu. F = 146 °C.

Analyse : $C_{12}H_{14}N_2O_2S$

| | | | | |
|---|---|---|---|---|
| Calculé : | C 57,60 | H 5,65 | N 11,20 | S 12,80 % |
| Trouvé : | C 57,65 | H 5,65 | N 11,20 | S 12,95 % |

Préparation B.

On ajoute sous agitation 261 g d'isocyanate de potassium à une solution de 154,6 g de 2-(p-aminophényl)propionate d'éthyle (préparé selon ARZNEIM.FORSCH 23,1090 (1973)) dans l'acide acétique, puis ajoute à 18°/25° C en 2 heures, une solution de 149,4 g de brome dans 750 $cm^3$ d'acide acétique, maintient sous agitation 30 minutes puis verse dans l'eau, filtre et amène à pH 5-6 à l'aide d'une solution aqueuse à 20 % de bicarbonate de soude. On extrait au chlorure de méthylène, sépare la phase organique, la lave à l'eau, la sèche et concentre à sec. On dissout le résidu dans 300 $cm^3$ d'acétate d'éthyle chaud, ajoute 300 $cm^3$ d'éther de pétrole (Eb. 40-60°) et laisse refroidir. On filtre le précipité, le lave, le sèche et recueille 97 g de produit attendu. F = 146 °C. Des eaux-mères, on récupère 29 g de produit supplémentaire.

Exemple 3 : 2-oxo 4-phényl 2H-pyrimido[2,1-b]benzothiazole 8-acétate d'éthyle.

On chauffe 1 heure sous agitation à 200 °C, 5 g de 2-amino benzothiazole 6-acétate d'éthyle et 6 g de phénylpropiolate d'éthyle, refrolidit et chromatographie le mélange sur silice en éluant au chloroforme. Après cristallisation dans le chloroforme et recristallisation dans l'éthanol, on obtient 1,99 g de produit attendu.

Exemple 4 : 2-oxo 4-phényl 2H-pyrimido[2,1-B]benzothiazole 8-acétate de méthyle

En utilisant une méthode analogue à celle décrite à l'exemple 1 mais en partant du 2-amino benzothiazole 6-acétate de méthyle et du phényl propiolate d'éthyle, on a obtenu le produit attendu.

Exemple 5 : 2-oxo 4-phényl 2H-pyrimido [2,1-b] benzothiazole 8-acétate de n-propyle

En utilisant une méthode analogue à celle décrite à l'exemple 3 mais en partant du 2-amino benzothiazole 6-acétate de n-propyle et du phényl propiolate d'éthyle, on a obtenu le produit attendu.

Exemple 6 : 2-oxo 4-phényl 2H-pyrimido [2,1-b] benzothiazole 8-acétate d'isopropyle

En utilisant une méthode analogue à celle décrite à l'exemple 3 mais en partant du 2-amino benzothiazole 6-acétate d'isopropyle et du phényl propiolate d'éthyle, on a obtenu le produit attendu.

Exemple 7 : 2-oxo 4-phényl 2H-pyrimido [2,1-b] benzothiazole 8-acétate de n-butyle

En utilisant une méthode analogue à celle décrite à l'exemple 3 mais en partant du 2-amino benzothiazole 6-acétate de n-butyle et du phényl propiolate d'éthyle, on a obtenu le produit attendu.

Exemple 8 : Acide 2-oxo 4-phényl 2H-pyrimido [2,1-b] benzothiazole 8-acétique.

On agite à température ambiante pendant 16 heures, le mélange comprenant 2,47 g de produit obtenu comme à l'exemple 3, 100 $cm^3$ de méthanol et 2,47 g de bicarbonate de soude dissous dans 20 $cm^3$ d'eau. On évapore le méthanol, dilue le milieu réactionnel avec de l'eau puis acidifie à l'aide d'acide chlorhydrique concentré. On filtre le précipité formé, le sèche et le recristallise à 2 reprises dans le méthanol et obtient 1,72 g de produit attendu.

En utilisant une méthode analogue à celle décrite à l'exemple 3 mais en partant du 2-amino benzothiazole 6-acétate d'éthyle et de composés de formule III dans laquelle $R_4$ représente un radical éthyle et $R_2$ représente les valeurs indiquées dans le tableau I ci-après, on a préparé les produits des exemples 9 à 19.

Exemple 9 : 4-(4-méthoxyphényl) 2-oxo 2H-pyrimido [2,1-b] benzothiazole 8-acétate d'éthyle.

Exemple 10 : 4-(4-nitrophényl) 2-oxo 2H-pyrimido [2,1-b] benzothiazole 8-acétate d'éthyle.

Exemple 11 : 4-(4-chlorophényl) 2-oxo 2H-pyrimido [2,1-b] benzothiazole 8-acétate d'éthyle.

Exemple 12 : 4-(2-chlorophényl) 2-oxo 2H-pyrimido [2,1-b] benzothiazole 8-acétate d'éthyle.

Exemple 13 : 4-(4-méthylphényl) 2-oxo 2H-pyrimido [2,1-b] benzothiazole 8-acétate d'éthyle.

Exemple 14 : 4-(3-chlorophényl) 2-oxo 2H-pyrimido [2,1-b] benzothiazole 8-acétate d'éthyle.

Exemple 15 : 4-méthyl 2-oxo 2H-pyrimido [2,1-b] benzothiazole 8-acétate d'éthyle.

Exemple 16 : 4-éthyl 2-oxo 2H-pyrimido [2,1-b] benzothiazole 8-acétate d'éthyle.

Exemple 17 : 2-oxo 4n-propyl 2H-pyrimido [2,1-b] benzothiazole 8-acétate d'éthyle.

Exemple 18 : 4n-butyl 2-oxo 2H-pyrimido [2,1-b] benzothiazole 8-acétate d'éthyle.

Exemple 19 : 4-méthoxycarbonyl-2-oxo 2H-pyrimido [2,1-b] benzothiazole 8-acétate d'éthyle.

Exemple 20 : α-méthyl 2-oxo 4-phényl 2H-pyrimido [2,1-b] benzothiazole 8-acétate d'éthyle.

On chauffe à 100 °C sous agitation pendant 20 heures, un mélange comprenant 50 g de 2-amino α-méthyl benzothiazole 6-acétate d'éthyle préparé comme à l'exemple 2 et 34,9 g de phényl propiolate d'éthyle. On ajoute de nouveau 7 g de phényl propiolate d'éthyle et poursuit le chauffage pendant 96 heures. On ajoute lentement 500 $cm^3$ d'éther et porte 2 heures au reflux.

On laisse refroidir, amorce la cristallisation, glace, filtre le précipité, le lave à l'éther, le sèche et recueille 39 g de produit attendu.

Exemple 21 : Acide α-méthyl 2-oxo 4-phényl 2H-pyrimido [2,1-b] benzothiazole acétique.

En utilisant une méthode analogue à celle de l'exemple 8 mais en partant du α-méthyl 2-oxo 4-phényl 2H-pyrimido [2,1-b] benzothiazole 8-acétate d'éthyle préparé comme à l'exemple 20, on obtient le produit attendu.

En utilisant une méthode analogue à celle décrite à l'exemple 20 mais en partant du 2-amino α-méthyl benzothiazole 6-acétate d'éthyle et du produit de formule III appropriée dans laquelle $R_2$ a la signification indiquée dans le tableau I ci-après et $R_4$ représente un radical éthyle, on a préparé les produits des exemples 22 à 24.

Exemple 22 : α-méthyl 4-(4-chlorophényl) 2-oxo 2H-pyrimido [2,1-b] benzothiazole 8-acétate d'éthyle.

Exemple 23 : α-méthyl 4-(3-chlorophényl) 2-oxo 2H-pyrimido [2,1-b] benzothiazole 8-acétate d'éthyle.

Exemple 24 : α-méthyl 2-oxo 4n-propyl 2H-pyrimido [2,1-b] benzothiazole 8-acétate d'éthyle.

En utilisant une méthode analogue à cele des exemples précédents en partant des composés de formules II et III appropriées, on a préparé les produits des exemples 25 à 43.

Exemple 25 : 4-isopropyl 2-oxo 2H-pyrimido [2,1-b] benzothiazole 8-acétate d'éthyle.

Exemple 26 : 4-benzyl 2-oxo 2H-pyrimido [2,1-b] benzothiazole 8-acétate d'éthyle.

Exemple 27 : α-méthyl 4-benzyl 2-oxo 2H-pyrimido [2,1-b] benzothiazole 8-acétate d'éthyle.

Exemple 28 : α-méthyl 4-isopropyl 2-oxo 2H-pyrimido [2,1-b] benzothiazole 8-acétate d'éthyle.

Exemple 29 : α-éthyl 4-phényl 2-oxo 2H-pyrimido [2,1-b] benzothiazole 8-acétate d'éthyle.

Exemple 30 : 4-cyclohexyl 2-oxo 2H-pyrimido [2,1-b] benzothiazole 8-acétate d'éthyle.

Exemple 31 : α-méthyl 4-cyclohexyl 2-oxo 2H-pyrimido [2,1-b] benzothiazole 8-acétate d'éthyle.

Exemple 32 : α-propyl 4-phényl 2-oxo 2H-pyrimido [2,1-b] benzothiazole 8-acétate d'éthyle.

Exemple 33 : α-isopropyl 4-phényl 2-oxo 2H-pyrimido [2,1-b] benzothiazole 8-acétate d'éthyle.

Exemple 34 : α-butyl 4-phényl 2-oxo 2H-pyrimido [2,1 b] benzothiazole 8-acétate d'éthyle.

Exemple 35 : α-méthyl 4-phényl 2-oxo 2H-pyrimido [2,1-b] benzothiazole 8-acétate d'isopropyle.

Exemple 36 : α,α-diméthyl 4-phényl 2-oxo 2H-pyrimido [2,1-b] benzothiazole 8-acétate d'éthyle.

Exemple 37 : α-éthyl α-méthyl 4-phényl 2-oxo 2H-pyrimido [2,1-b] benzothiazole 8-acétate d'éthyle.

Exemple 38 : α-méthyl α-propyl 4-phényl 2-oxo 2H-pyrimido [2,1-b] benzothiazole 8-acétate d'éthyle.

Exemple 39 : α-méthyl 4-phényl 2-oxo 2H-pyrimido [2,1-b] benzothiazole 8-acétate de t-butyle.

Exemple 40 : α,α-diéthyl 4-phényl 2-oxo 2H-pyrimido [2,1-b] benzothiazole 8-acétate d'éthyle.

Exemple 41 : α-éthyl α-propyl 4-phényl 2-oxo 2H-pyrimido [2,1-b] benzothiazole 8-acétate d'éthyle.

Exemple 42 : α-butyl α-méthyl 4-phényl 2-oxo 2H-pyrimido [2,1-b] benzothiazole 8-acétate d'éthyle.

Exemple 43 : α-méthyl 4-phényl 2-oxo 2H-pyrimido [2,1-b] benzothiazole 8-acétate de 2,2-diméthylpropyle.

Exemple 44 : N,N-diméthyl 2-oxo 4-phényl 2H-pyrimido [2,1-b] benzothiazole 8-acétamide.

On ajoute 2 g de 1,1-carbonyl diimidazolole dans une solution de 3 g d'acide 2-oxo 4-phényl 2H-pyrimido [2,1-b] benzothiazole 8-acétique dans 40 $cm^3$ de tétrahydrofuranne, agite 1 heure à température ambiante, ajoute de la diméthylamine en excès à l'aide d'une solution éthanolique à 30 % et poursuit l'agitation pendant 40 minutes.

On élimine le tétrahydrofuranne par distillation sous pression réduite, reprend le résidu par du dichlorométhane, lave à l'eau avec une solution aqueuse de bicarbonate de soude puis de nouveau à l'eau sèche et concentre à sec sous pression réduite. On purifie le produit brut par chromatographie sur silice et obtient 1,1 g de produit attendu.

En opérant selon les méthodes analogues à celles décrites précédemment en utilisant au départ le composé aminé approprié, on a préparé les produits des exemples 45 à 48.

Exemple 45 : N-éthyl 2-oxo 4-phényl 2H-pyrimido [2,1-b] benzothiazole 8-acétamide.

Exemple 46 : 1-(N-morpholino)-2-(2-oxo 4-phényl 2H-pyrimido [2,1-b] benzothiazole 8-yl) éthanone.

Exemple 47 : N,N-diéthyl 2-oxo 4-phényl 2H-pyrimido [2,1-b] benzothiazole 8-acétamide.

Exemple 48 : 1-(N-pipéridino)-2-(2-oxo 4-phényl 2H-pyrimido [2,1-b] benzothiazole 8-yl] éthanone.

Les résultats d'analyse et les points de fusion des produits des exemples 1 à 48 sont donnés dans le tableau 1 ci-après.

(Voir Tableau 1 pages 11 et s.)

Tableau 1

| Exemple | R | $R_1$ | $R_2$ | $R_3$ | F°C |
|---|---|---|---|---|---|
| 1 | H | EtO | H | H | 276 |
| 2 | Me | EtO | H | H | 129 |
| 3 | H | EtO | Ph | H | 206 |
| 4 | H | MeO | Ph | H | 225-6 |
| 5 | H | nPrO | Ph | H | 133.5- |
| | | | | H | 134.5 |
| 6 | H | isoPrO | Ph | H | 171.5-172.5 |
| 7 | H | nBuO | Ph | H | 136-7 |
| 8 | H | HO | Ph | H | slight decompos. 136°C m.256°C |
| 9 | H | EtO | p-MeOPh | H | 152 |
| 10 | H | EtO | p-$NO_2$Ph | H | 246 |
| 11 | H | EtO | p-ClPh | H | 220 |
| 12 | H | EtO | o-ClPh | H | 196.5 |
| 13 | H | EtO | p-MePh | H | 183-4 |
| 14 | H | EtO | m-ClPh | H | 176-9 |
| 15 | H | EtO | Me | H | 251-3 |
| 16 | H | EtO | Et | H | 166.7 |
| 17 | H | EtO | nPr | H | 158-60 |
| 18 | H | EtO | nBu | H | 149.6 |
| 19 | H | EtO | $CO_2$Me | H | 163 |
| 20 | Me | EtO | Ph | H | 127-31 |
| 21 | Me | HO | Ph | H | 239-46 |
| 22 | Me | EtO | p-ClPh | H | 190-1 |
| 23 | Me | EtO | m-ClPh | H | 76-9 |
| 24 | Me | EtO | nPr | H | 125-6 |
| 25 | H | EtO | isoPr | H | 170-2 |
| 26 | H | EtO | $CH_2$Ph | H | 197-9 |
| 27 | Me | EtO | $CH_2$Ph | H | 99-101 |
| 28 | Me | EtO | isoPr | H | 149-51 |
| 29 | Et | EtO | Ph | H | 143-5 |
| 30 | H | EtO | cyclohexyl | H | 169-71 |
| 31 | Me | EtO | cyclohexyl | H | 148-50 |
| 32 | nPr | EtO | Ph | H | 131-3 |
| 33 | isoPr | EtO | Ph | H | 126-8 |
| 34 | Bu | EtO | Ph | H | 92-94 |

Tableau 1 (Suite)

| Exemple | R | $R_1$ | $R_2$ | $R_3$ | F°C |
|---|---|---|---|---|---|
| 35 | Me | isoPrO | Ph | H | 153-5 |
| 36 | Me | EtO | Ph | Me | 133-5 |
| 37 | Me | EtO | Ph | Et | 159-61 |
| 38 | Me | EtO | Ph | Pr | 174-6 |
| 39 | Me | t-BuO | Ph | H | 122-5 |
| 40 | Et | EtO | Ph | Et | 138-40 |
| 41 | Et | EtO | Ph | Pr | 163-4 |
| 42 | Me | EtO | Ph | Bu | 143-4 |
| 43 | Me | t-$BuCH_2O$ | Ph | H | 122-3 |
| 44 | H | $Me_2N$ | Ph | H | 238-40 |
| 45 | H | EtHN | Ph | H | 210-2 |
| 46 | H | 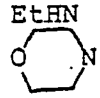 | Ph | H | 214-6 |
| 47 | H | $Et_2N$ | Ph | H | 147-9 |
| 48 | H | 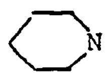 | Ph | H | 190-1 |

Tableau 1 (Suite)

| Exemple | Formule | Calculé %C | %H | N% | S% |
|---|---|---|---|---|---|
| 1 | $C_{14}H_{12}N_2O_3S$ | 58.3 | 4.2 | 9.7 | 11.1 |
| 2 | $C_{15}H_{14}N_2O_3S$ | 59.6 | 4.65 | 9.25 | 10.6 |
| 3 | $C_{20}H_{16}N_2O_3S$ | 65.9 | 4.4 | 7.7 | 8.8 |
| 4 | - | - | - | - | - |
| 5 | $C_{21}H_{18}N_2O_3S$ | 66.65 | 4.8 | 7.4 | 8.45 |
| 6 | $C_{21}H_{18}N_2O_3S$ | 66.65 | 4.8 | 7.4 | 8.45 |
| 7 | $C_{22}H_{19}N_2O_3S$ | 67.35 | 5.15 | 7.15 | 8.15 |
| 8 | $C_{18}H_{12}N_2O_3S$ | 64.25 | 3.6 | 8.35 | 9.55 |
| 9 | $C_{21}H_{18}N_2O_4S$ | 63.95 | 4.6 | 7.1 | 8.15 |
| 10 | $C_{20}H_{15}N_3O_5S$ | 58.65 | 3.7 | 10.25 | 7.85 |
| 11 | $C_{20}H_{15}N_2O_3ClS$ | 60.2 | 3.8 | 7.0 | 8.05 |
| 12 | $C_{20}H_{15}N_2O_3ClS$ | 60.2 | 3.8 | 7.0 | 8.05 |
| 13 | $C_{21}H_{18}N_2O_3S$ | 66.65 | 4.8 | 7.4 | 8.45 |
| 14 | $C_{20}H_{15}N_2O_3ClS$ | 60.2 | 3.8 | 7.0 | 8.05 |
| 15 | $C_{15}H_{14}N_2O_3S$ | 59.6 | 4.65 | 9.25 | 10.6 |
| 16 | $C_{16}H_{16}N_2O_3S$ | 60.75 | 5.1 | 8.85 | 10.15 |
| 17 | $C_{17}H_{18}N_2O_3S$ | 61.8 | 5.5 | 8.5 | 9.7 |

Tableau 1 (Suite)

| | | Calculé | | | |
|---|---|---|---|---|---|
| Exemple | Formule | %C | %H | N% | S% |
| 18 | $C_{18}H_{20}N_2O_3S$ | 62.75 | 5.85 | 8.15 | 9.3 |
| 19 | $C_{16}H_{14}N_2O_5S$ | 55.5 | 4.05 | 8.1 | 9.25 |
| 20 | $C_{21}H_{18}N_2O_3S$ | 66.65 | 4.8 | 7.4 | 8.45 |
| 21 | $C_{19}H_{14}N_2O_3S$ | 66.15 | 4.05 | 8.0 | 9.15 |
| 22 | $C_{21}H_{17}N_2O_3ClS$ | 61.1 | 4.15 | 6.8 | 7.75 |
| 23 | $C_{21}H_{17}N_2O_3ClS$ | 61.1 | 4.15 | 6.8 | 7.75 |
| 24 | $C_{18}H_{20}N_2O_3S$ | 62.75 | 5.85 | 8.15 | 9.3 |
| 25 | $C_{17}H_{18}N_2O_3S$ | 61.80 | 5.49 | 8.48 | 9.70 |
| 26 | $C_{21}H_{18}N_2O_3S$ | 66.65 | 4.79 | 7.40 | 8.47 |
| 27 | $C_{22}H_{20}N_2O_3S.1/2H_2O$ | 65.82 | 5.27 | 6.98 | 7.99 |
| 28 | $C_{18}H_{20}N_2O_3S$ | 62.77 | 5.85 | 8.13 | 9.29 |
| 29 | $C_{22}H_{20}N_2O_3S$ | 67.33 | 5.14 | 7.14 | 8.17 |
| 30 | $C_{20}H_{22}N_2O_3S$ | 64.84 | 5.99 | 7.56 | 8.65 |
| 31 | $C_{21}H_{24}N_2O_3S$ | 65.60 | 6.29 | 7.28 | 8.34 |
| 32 | $C_{23}H_{22}N_2O_3S$ | 67.96 | 5.46 | 6.89 | 7.89 |
| 33 | $C_{23}H_{22}N_2O_3S.1/2H_2O$ | 66.49 | 5.58 | 6.74 | 7.72 |
| 34 | $C_{24}H_{24}N_2O_3S$ | 68.55 | 5.75 | 6.66 | 7.62 |
| 35 | $C_{22}H_{20}N_2O_3S$ | 67.33 | 5.14 | 7.14 | 8.17 |
| 36 | $C_{22}H_{20}N_2O_3S$ | 67.33 | 5.14 | 7.14 | 8.17 |
| 37 | $C_{23}H_{22}N_2O_3S$ | 67.96 | 5.46 | 6.89 | 7.89 |
| 38 | $C_{24}H_{24}N_2O_3S$ | 68.55 | 5.75 | 6.66 | 7.62 |
| 39 | $C_{23}H_{22}N_2O_3S$ | 67.96 | 5.46 | 6.89 | 7.89 |
| 40 | $C_{24}H_{24}N_2O_3S$ | 68.55 | 5.75 | 6.66 | 7.62 |
| 41 | $C_{25}H_{26}N_2O_3S$ | 69.10 | 6.03 | 6.45 | 7.38 |
| 42 | $C_{25}H_{26}N_2O_3S$ | 69.10 | 6.03 | 6.45 | 7.38 |
| 43 | $C_{24}H_{24}N_2O_3S$ | 68.55 | 5.75 | 6.66 | 7.62 |
| 44 | $C_{20}H_{17}N_3O_2S.1/2H_2O$ | 64.50 | 4.87 | 11.28 | 8.61 |
| 45 | $C_{20}H_{17}N_3O_2S.1/2H_2O$ | 64.50 | 4.87 | 11.28 | 8.61 |
| 46 | $C_{22}H_{19}N_3O_3S.1/2H_2O$ | 63.75 | 4.86 | 10.14 | 7.73 |
| 47 | $C_{22}H_{21}N_3O_2S$ | 67.50 | 5.41 | 10.73 | 8.19 |
| 48 | $C_{23}H_{21}N_3O_2S$ | 68.45 | 5.25 | 10.41 | 7.95 |

Tableau 1 (Suite)

Trouvé

| Exemple | %C | %H | %N | %S |
|---|---|---|---|---|
| 1 | 58.25 | 4.25 | 9.75 | 11.1 |
| 2 | 59.5 | 4.65 | 9.3 | 10.45 |
| 3 | 66.2 | 4.5 | 7.65 | 8.85 |
| 4 | - | - | - | - |
| 5 | 66.55 | 4.85 | 7.35 | 8.35 |
| 6 | 66.65 | 4.85 | 7.4 | 8.45 |
| 7 | 67.35 | 5.15 | 7.2 | 8.2 |
| 8 | 64.25 | 3.65 | 8.3 | 9.55 |
| 9 | 64.05 | 4.65 | 7.1 | 8.2 |
| 10 | 58.6 | 3.75 | 10.25 | 7.8 |
| 11 | 60.15 | 3.85 | 6.95 | 8.05 |
| 12 | 60.25 | 3.9 | 7.0 | 8.1 |
| 13 | 66.45 | 4.85 | 7.40 | 8.6 |
| 14 | 60.25 | 3.9 | 7.05 | 8.1 |
| 15 | 59.6 | 4.7 | 9.3 | 10.55 |
| 16 | 60.5 | 5.1 | 8.8 | 10.15 |
| 17 | 61.7 | 5.5 | 8.45 | 9.75 |
| 18 | 62.5 | 5.9 | 8.15 | 9.45 |
| 19 | 55.3 | 4.15 | 8.0 | 9.15 |
| 20 | 66.5 | 4.85 | 7.45 | 8.4 |
| 21 | 64.9 | 4.05 | 8.0 | 9.1 |
| 22 | 61.1 | 4.25 | 6.75 | 7.75 |
| 23 | 60.85 | 4.3 | 6.75 | 7.85 |
| 24 | 62.65 | 5.85 | 8.15 | 9.3 |

Tableau 1 (Suite)

| Exemple | Trouvé %C | %H | %N | %S |
|---|---|---|---|---|
| 25 | 61.88 | 5.51 | 8.51 | 9.64 |
| 26 | 66.55 | 4.85 | 7.37 | 8.43 |
| 27 | 65.94 | 5.34 | 6.88 | 8.02 |
| 28 | 62.73 | 5.93 | 8.11 | 9.17 |
| 29 | 67.43 | 5.16 | 7.14 | 8.12 |
| 30 | 64.85 | 5.95 | 7.62 | 8.64 |
| 31 | 65.62 | 6.34 | 7.21 | 8.35 |
| 32 | 67.84 | 5.54 | 6.85 | 7.75 |
| 33 | 66.56 | 5.59 | 6.61 | 7.65 |
| 34 | 68.35 | 5.83 | 6.62 | 7.72 |
| 35 | 67.27 | 5.20 | 7.08 | 7.97 |
| 36 | 67.42 | 5.16 | 7.09 | 8.22 |
| 37 | 67.66 | 5.66 | 6.67 | 7.59 |
| 38 | 68.30 | 5.86 | 6.54 | 7.53 |
| 39 | 68.10 | 5.50 | 6.84 | 7.87 |
| 40 | 68.47 | 5.85 | 6.58 | 7.61 |
| 41 | 69.08 | 6.14 | 6.34 | 7.37 |
| 42 | 68.91 | 6.14 | 6.32 | 7.28 |
| 43 | 68.62 | 5.78 | 6.66 | 7.63 |
| 44 | 64.50 | 4.72 | 11.18 | 8.54 |
| 45 | 64.46 | 4.71 | 11.28 | 8.62 |
| 46 | 63.86 | 4.76 | 10.00 | 7.75 |
| 47 | 67.20 | 5.40 | 10.58 | 8.17 |
| 48 | 68.19 | 5.31 | 10.26 | 7.95 |

Exemple 49

On a préparé des comprimés répondant à la formulation suivante :

| | |
|---|---|
| Produit de l'exemple 3 | 15 mg |
| Excipient q.s. pour un comprimé terminé à | 100 mg |

(Détail de l'excipient : lactose, amidon, talc, stéarate de magnésium).

Exemple 50

On a préparé un aérosol délivrant par dose :

| | |
|---|---|
| Produit de l'exemple 20 | 2 mg |
| Emulsifiant | 0,15 mg |
| Propulseur | 50 mg |

Exemple 51

On a préparé des comprimés répondant à la formulation suivante :

| | |
|---|---|
| Produit de l'exemple 20 | 15 mg |
| Excipient q.s. pour un comprimé terminé à | 100 mg |

(Détail de l'excipient : lactose, amidon, talc, stéarate de magnésium).

Etude pharmacologique

Augmentation de la pression de perfusion dans les poumons induite par un antigène.

On utilise pour ce test des cochons d'Inde mâles Dunkin Harley (Porcellus) pesant 450 à 700 g (à raison de 4 animaux par dose se produit à tester).

Les animaux sont sensibilisés par exposition bihebdomadaire à un aérosol contenant de l'ovalbumine (1 % poids/volume).

Après avoir été anesthésiés au moyen d'une injection intra-péritonéale de diazépam (2,5 mg/kg) et d'une injection intra-musculaire d'hypnorm (1 ml/kg), les animaux sont exsanguinés par section des carotides.

On ouvre la cage thoracique, prélève les poumons, les partage en 2 (au niveau du sternum) et les relie à un système de perfusion après avoir introduit une canule dans une bronche du lobe principal.

Les poumons sont perfusés avec du liquide de Krebs oxygène (95 % d'oxygène — 5 % de gaz carbonique) à 37 °C.

On injecte une solution de l'ovalbumine à raison de 5 µg pour 0,1 ml à proximité de chaque poumon. On enregistre l'augmentation de la pression de la perfusion due à l'antigène.

60 minutes plus tard, on administre 15 µg d'ovalbumine. Les produits à tester sont ajoutés au liquide de Krebs 30 minutes avant la 2[e] administration d'antigène.

Lors de chaque série de traitements, on effectue des mesures de contrôle sur des animaux témoins ne recevant aucun produit test (n =10).

La réponse au second antigène administré, est exprimée en pourcentage du premier.

Pour chaque administration de produit à tester, on utilise au moins 4 poumons (de 4 animaux différents, on calcule le pourcentage d'inhibition de la bronchoconstriction induite par l'antigène. ($IC_{50}$ en µM).

Les résultats sont donnés dans le Tableau 2 ci-dessous.

Tableau 2

| Produit de l'exemple | $CI_{50}$ µM |
|---|---|
| 2 | $>100$ |
| 3 | 1 |
| 4 | 10 |
| 5 | $<10$ |
| 6 | 1-10 |
| 7 | $>10$ |
| 9 | 1 |
| 10 | 10-100 |
| 11 | 10 |
| 12 | 0.1-1 |
| 13 | $<1$ |
| 14 | $\leq 1$ |
| 15 | 10-100 |
| 16 | 100 |
| 17 | 1-10 |
| 18 | 10 |

Tableau 2 (suite)

| Produit de l'exemple | $CI_{50}$ µM |
|---|---|
| 20 | 0.1 |
| 22 | 0.1-1 |
| 23 | ≤ 1 |
| 24 | > 10 |
| 25 | 10-100 |
| 27 | 10-100 |
| 28 | 10-100 |
| 29 | 0.1 |
| 30 | 1-10 |
| 31 | < 10 |
| 32 | 1 |
| 33 | 10-100 |
| 34 | 1-10 |
| 35 | 0.1-1 |
| 36 | 1-10 |
| 37 | 1 |
| 38 | 1 |
| 39 | 0.1 |
| 40 | 10-100 |
| 41 | 10-100 |
| 42 | 10-100 |
| 43 | 0.1 |
| 44 | 10-100 |
| 45 | 10-100 |
| 46 | > 100 |
| 47 | 10 |
| 48 | 10-100 |

**Revendications**

1. Dérivés du pyrimido [2,1-b] benzothiazole ainsi que leurs sels, caractérisés en ce qu'ils répondent à la formule générale (I) :

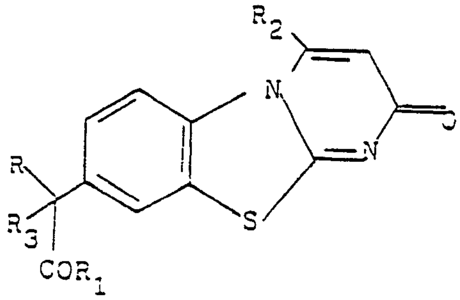

(I)

dans laquelle R et $R_3$, identiques ou différents, représentent un atome d'hydrogène, un radical alcoyle, linéaire ou ramifié, renfermant de 1 à 6 atomes de carbone ou R et $R_3$ forment avec l'atome de carbone auquel ils sont liés, un radical cycloalcoyle renfermant de 3 à 6 atomes de carbone, $R_1$ représente un radical hydroxy, un radical alcoxy, linéaire ou ramifié, renfermant de 1 à 6 atomes de carbone ou un radical

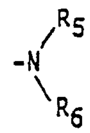

dans lequel $R_5$ et $R_6$, identiques ou différents, représentent un atome d'hydrogène, un radical alcoyle renfermant de 1 à 6 atomes de carbone ou $R_5$ et $R_6$ forment avec l'atome d'azote auquel ils sont liés un radical pipéridino ou morpholino, $R_2$ représente un atome d'hydrogène, un radical alcoyle, linéaire ou ramifié, renfermant de 1 à 6 atomes de carbone, un radical aryle renfermant de 6 à 12 atomes de carbone, un radical aryle renfermant de 6 à 12 atomes de carbone substitué par un ou plusieurs radicaux alcoyles ou alcoxy renfermant de 1 à 6 atomes de carbone, par un ou plusieurs atomes d'halogène ou par un ou plusieurs radicaux nitro, ou $R_2$ représente un radical 2-thiényle, un radical aralcoyle renfermant de 7 à 13 atomes de carbone, un radical alcoxy carbonyle renfermant de 2 à 7 atomes de carbone ou un radical cycloalcoyle renfermant de 3 à 6 atomes de carbone.

2. Dérivés répondant à la formule générale (I) de la revendication 1, ainsi que leurs sels, caractérisés en ce que dans ladite formule (I), R et $R_3$ représentent un atome d'hydrogène ou un radical méthyle ou éthyle, $R_1$ et $R_2$ ont la signification déjà indiquée.

3. Dérivés répondant à la formule générale (I) de la revendication 1, ainsi que de leurs sels, caractérisés en ce que dans ladite formule (I), R et $R_3$ représentent un atome d'hydrogène ou un radical méthyle ou éthyle, $R_1$ a la signification déjà indiquée, $R_2$ représente un atome d'hydrogène, un radical alcoyle renfermant de 1 à 6 atomes de carbone, un radical phényle, un radical phényle substitué par un ou plusieurs radicaux méthyle, par un ou plusieurs atomes d'halogène, ou par un ou plusieurs radicaux nitro ou méthoxy ou $R_2$ représente un radical méthoxy carbonyle.

4. L'un quelconque des produits de formule (I) telle que définie à la revendication 1, dont les noms suivent :

— le 2-oxo 4-phényl 2H-pyrimido [2,1-b] benzothiazole 8-acétate d'éthyle,
— le 2-oxo 4-(p-méthoxy phényl) 2H-pyrimido [2,1-b] benzothiazole 8-acétate d'éthyle,
— le 2-oxo 4-(o-chlorophényl) 2H-pyrimido [2,1-b] benzothiazole 8-acétate d'éthyle,
— l'α-méthyl 2-oxo 4-phényl 2H-pyrimido [2,1-b] benzothiazole 8-acétate d'éthyle,
— l'α-méthyl 2-oxo 4-(p-chlorophényl) 2H-pyrimido [2,1-b] benzothiazole 8-acétate d'éthyle,
— l'α-éthyl 2-oxo 4-phényl 2H-pyrimido [2,1-b] benzothiazole 8-acétate d'éthyle,

ainsi que leurs sels d'addition avec les acides.

5. Procédé de préparation des nouveaux dérivés du pyrimido [2,1-b] benzothiazole ainsi que de leurs sels, caractérisé en ce que l'on fait réagir un produit de formule (II) :

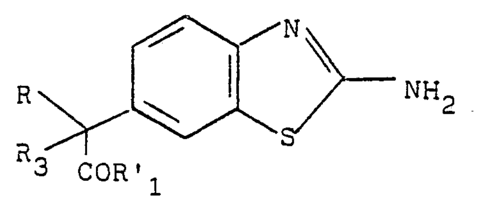

dans laquelle R et $R_3$ ont la signification indiquée à la revendication 1, $R'_1$ représente un radical hydroxy ou un radical alcoxy renfermant de 1 à 6 atomes de carbone avec un produit de formule (III) :

$$R_2—C≡C—CO_2R_4 \qquad \text{(III)}$$

dans laquelle $R_2$ a la signification déjà indiquée et $R_4$ représente un radical alcoyle renfermant de 1 à 3 atomes de carbone, pour obtenir un produit de formule ($I_A$) :

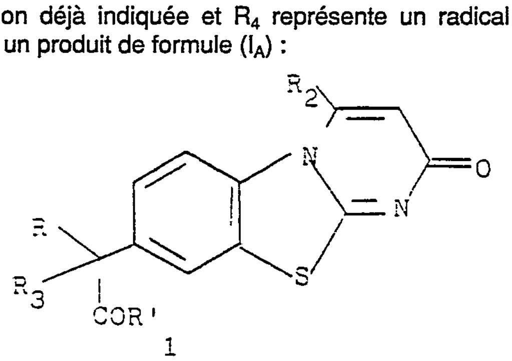

(IA)

dans laquelle R, $R_3$, R'1 et $R_2$ ont la signification déjà indiquée et que :

soit on isole et, si désiré, salifie le produit de formule (I) ainsi 15 obtenu ;

soit on saponifie un produit de formule ($I_A$) ainsi obtenu dans laquelle $R'_1$ représente un radical alcoxy, pour obtenir un produit de formule ($I_B$) :

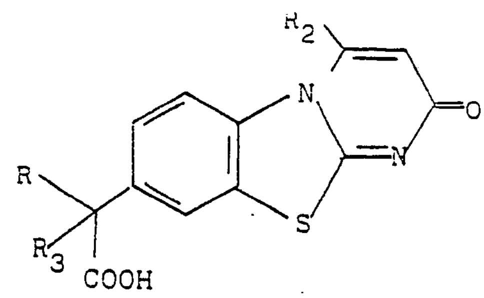

(IB)

dans laquelle R, $R_3$ et $R_2$ ont la signification déjà indiquée que, le cas échéant, l'on isole et, si désiré, salifie ou que l'on estérifie par réaction avec un alcool de formule (IV) :

$$HO—R''_1 \quad (IV)$$

dans laquelle $R''_1$ représente un radical alcoyle renfermant de 1 à 6 atomes de carbone, pour obtenir un produit de formule (I) correspondant dans laquelle $R_1$ a la signification de $R''_1$ et, si désiré, salifie ce dernier ;

soit on transestérifie un produit de formule ($I_A$) dans laquelle $R'_1$ représente un radical alcoxy, par action d'un alcool de formule (IV) :

$$HO—R''_1 \quad (IV)$$

dans laquelle $R''_1$ a la signification déjà indiquée, pour obtenir un produit de formule (I) correspondant et, si désiré, salifie ce dernier ;

soit on fait réagir un produit de formule ($I_A$) dans laquelle $R'_1$ représente un radical hydroxy, avec un produit de formule :

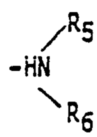

dans laquelle $R_5$ et $R_6$ ont la signification déjà indiquée, pour obtenir un produit de formule (I) correspondant et, si désiré, salifie ce dernier.

6. Procédé de préparation des nouveaux dérivés du pyrimido [2,1-b] benzothiazole tel que défini à la revendication 5, caractérisé en ce que :

a) la réaction du produit de formule (II) avec le produit de formule (III) est effectuée dans le cas où $R_2$ représente un atome d'hydrogène, au reflux du mélange réactionnel, au sein d'un alcanol tel que l'éthanol et en présence d'un catalyseur tel que le palladium ;

b) la réaction du produit de formule (II) avec le produit de formule (III) est effectuée, dans le cas où $R_2$ ne représente pas un atome d'hydrogène, à une température de 100 à 180 °C, en l'absence de solvant ;

c) la saponification du produit de formule ($I_A$) est effectuée au moyen d'une base faible telle que le carbonate de potassium ;

d) la réaction du produit de formule ($I_A$) avec le produit de formule : $HNR_5R_6$ est effectuée en présence de 1,1'-carbodiimidazole.

7. Médicaments, caractérisés en ce qu'ils sont constitués par les nouveaux dérivés du pyrimido [2,1-b] benzothiazole tels que définis par la formule (I) de la revendication 1 ainsi que par leurs sels d'addition avec les bases et les acides pharmaceutiquement acceptables.

8. Médicaments, caractérisés en ce qu'ils sont constitués par les nouveaux dérivés tels que définis à la revendication 2, 3 ou 4 ainsi que par leurs sels d'addition avec les bases et les acides pharmaceutiquement acceptables.

9. Compositions pharmaceutiques, caractérisées en ce qu'elles renferment à titre de principe actif, l'un au moins des médicaments tels que définis à l'une quelconque des revendications 7 ou 8.

10. Les produits de formule (II) :

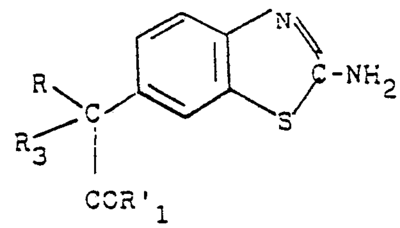

(II)

dans laquelle R, $R_3$ et $R'_1$ ont la signification indiquée à la revendication 5, étant entendu que lorsque $R'_1$ représente un radical hydroxy, le groupement $R_3RC<$ est différent de $H_3C—CH<$ ou de $H_2C<$ et que lorsque $R'_1$ représente un radical éthoxy, le groupement $R_3RC<$ est différent de $H_2C<$.

## Claims

1. Derivatives of pyrimido [2,1-b] benzothiazole as well as their salts, characterized in that they correspond to the general formula (I) :

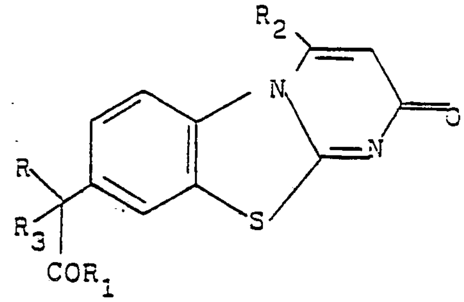

(I)

in which R and $R_3$, identical or different, each represents a hydrogen atom, a linear or branched alkyl radical containing from 1 to 6 carbon atoms or R and $R_3$ form, together with the carbon atom to which they are joined, a cycloalkyl radical containing from 3 to 6 carbon atoms, $R_1$ represents a hydroxy radical, a linear or branched alkoxy radical containing from 1 to 6 carbon atoms or a radical :

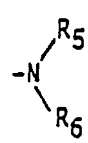

in which $R_5$ and $R_6$, identical or different, each represents a hydrogen atom, or an alkyl radical containing from 1 to 6 carbon atoms, or $R_5$ and $R_6$ form, together with the nitrogen atom to which they are joined, a piperidino or morpholino radical, $R_2$ represents a hydrogen atom, a linear or branched alkyl radical containing from 1 to 6 carbon atoms, an aryl radical containing from 6 to 12 carbon atoms, an aryl radical containing from 6 to 12 carbon atoms substituted by one or more alkyl or alkoxy radicals containing from 1 to 6 carbon atoms, by one or more halogen atoms or by one or more nitro radicals, or $R_2$ represents a 2-thienyl radical, an aralkyl radical containing from 7 to 13 carbon atoms, an alkoxy carbonyl radical containing from 2 to 7 carbon atoms or a cycloalkyl radical containing from 3 to 6 carbon atoms.

2. Derivatives corresponding to the general formula (I) of claim 1, as well as their salts, characterized in that in the said formula (I), R and $R_3$ represent a hydrogen atom or a methyl or ethyl radical, and $R_1$ and $R_2$ have the significance already indicated.

3. Derivatives corresponding to the general formula (I) of claim 1, as well as their salts, characterized in that in the said formula (I), R and $R_3$ represent a hydrogen atom or a methyl or ethyl radical, $R_1$ has the significance already indicated, $R_2$ represents a hydrogen atom, an alkyl radical containing from 1 to 6 carbon atoms, a phenyl radical, a phenyl radical substituted by one or more methyl radicals, by one or more halogen atoms, or by one or more nitro or methoxy radicals, or $R_2$ represents a methoxy carbonyl radical.

4. Any one of the products of formula (I) as defined in claim 1, of which th the names follow :

Ethyl 2-oxo-4-phenyl-2H-pyrimido [2,1-b] benzothiazole-8-acetate
Ethyl 2-oxo-4-(p-methoxyphenyl)-2H-pyrimido [2,1-b] benzothiazole-8-acetate
Ethyl 2-oxo-4-(o-chlorophenyl)-2H-pyrimido [2,1-b] benzothiazole-8-acetate
Ethyl l'alpha-methyl-2-oxo-4-phenyl-2H-pyrimido [2,1-b] benzothiazole-8-acetate

Ethyl l'alpha-methyl-2-oxo-4-(p-chlorophenyl)-2H-pyrimido [2,1-b]-benzothiazole-8-acetate
Ethyl l'alpha-ethyl-2-oxo-4-phenyl-2H-pyrimido [2,1-b] benzothiazole-8-acetate,

as well as their addition salts with acids.

5. Preparation process for new derivatives of pyramido [2,1-b] benzothiazole, as well as their salts, characterized in that a product of general formula (II) :

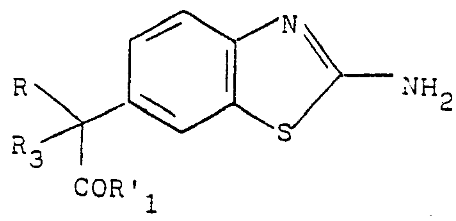

(II)

in which R and $R_3$ have the significance indicated in claim 1, $R'_1$ represents a hydroxy radical or an alkoxy radical containing from 1 to 6 carbon atoms, is reacted with a product of formula (III) :

$$R_2—C{\equiv}C—CO_2R_4 \quad \text{(III)}$$

in which $R_2$ has the significance already indicated and $R_4$ represents an alkyl radical containing from 1 to 3 carbon atoms, so as to obtain a product of formula ($I_A$) :

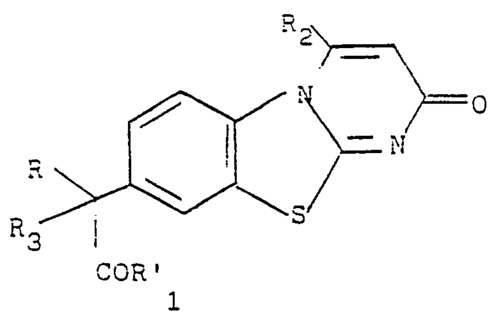

(IA)

in which R, $R_3$, $R'_1$ and $R_2$ have the significance already indicated and that :

either the product of formula (I) thus obtained is isolated and, if desired, salified ;

or a product of formula ($I_A$) thus obtained, in which $R'_1$ represents an alkoxy radical, is saponified so as to obtain a product of formula ($I_B$) :

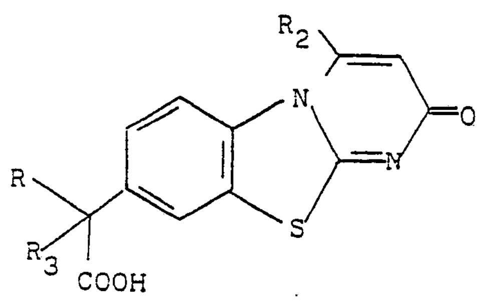

(IB)

in which R, $R_3$ and $R_2$ have the significance already indicated, which, if appropriate, is isolated and, if desired, salified or which is esterified by reaction with an alcohol of formula (IV) :

$$HO—R''_1 \quad \text{(IV)}$$

in which $R''_1$ represents an alkyl radical containing from 1 to 6 carbon atoms, so as to obtain a corresponding product of formula (I) in which $R_1$ has the significance of $R''_1$ and, if desired, the latter is salified ;

or a product of formula ($I_A$) in which $R'_1$ represents an alkoxy radical is transesterified by the action of an alcohol of formula (IV) :

$$HO—R''_1 \quad \text{(IV)}$$

in which $R''_1$ has the significance already indicated, so as to obtain a corresponding product of formula (I) and, if desired, the latter is salified ;

or a product of formula ($I_A$) in which $R'_1$ represents a hydroxy radical is reacted with a product of formula :

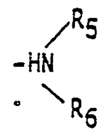

in which $R_5$ and $R_6$ have the significance already indicated, so as to obtain a corresponding product of formula (I) and, if desired, the latter is salified.

6. Preparation process for new derivatives of pyrimido [2,1-b] benzothiazole as defined in claim 5, characterized in that :

a) the reaction of the product of formula (II) with the product of formula (III) is carried out, when $R_2$ represents a hydrogen atom, at reflux of the reaction mixture, in an alkanol such as ethanol and in the presence of a catalyst such as palladium ;

b) the reaction of the product of formula (II) with the product of formula (III) is carried out, when $R_2$ does not represent a hydrogen atom, at a temperature of 100 to 180 °C, without a solvent ;

c) the saponification of the product of formula ($I_A$) is carried out using a weak base such as potassium carbonate ;

d) the reaction of the product of formula ($I_A$) with the product of formula $HNR_5R_6$ is carried out in the presence of 1,1'-carbodiimidazole.

7. Medicaments, characterized in that they are made up of the new derivatives of pyrimido [2,1-b] benzothiazole as defined by the formula (I) of claim 1, as well as of their pharmaceutically acceptable addition salts with bases and acids.

8. Medicaments, characterized in that they are made up of the new derivatives as defined in claim 2, 3 or 4, as well as of their pharmaceutically acceptable addition salts with bases and acids.

9. Pharmaceutical compositions, characterized in that they contain, as active principle, at least one of the medicaments as defined in any one of claims 7 or 8.

10. The products of formula (II) :

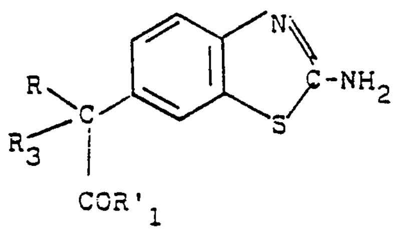

(II)

in which R, $R_3$ and $R'_1$ have the significance indicated in claim 5, it being understood that when $R'_1$ represents a hydroxy radical, the group $R_3R_C\leq$ is different from $H_3C-CH<$ or from $H_2C<$ and that when $R'_1$ represents an ethoxy radical, the group $R_3RC\leq$ is different from $H_2C\leq$.

## Patentansprüche

1. Pyrimido (2,1-b) benzothiazolderivate sowie deren Salze, dadurch gekennzeichnet, daß sie der allgemeinen Formel (I)

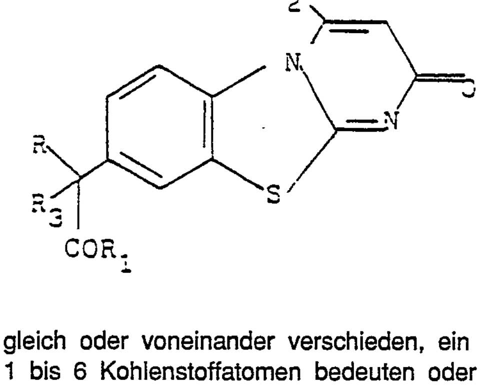

(I)

entsprechen, worin R und $R_3$, gleich oder voneinander verschieden, ein Wasserstoffatom, einen linearen oder verzweigten Alkylrest mit 1 bis 6 Kohlenstoffatomen bedeuten oder R und $R_5$ mit dem Kohlenstoff-

atom, an das sie gebunden sind, einen Cycloalkylrest mit 3 bis 6 Kohlenstoffatomen bilden, $R_1$ für eine Hydroxygruppe, eine lineare oder verzweigte Alkoxygruppe mit 1 bis 6 Kohlenstoffatomen oder einen Rest

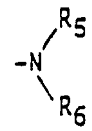

steht, worin $R_5$ und $R_6$, gleich oder voneinander verschieden, ein Wasserstoffatom oder einen Alkylrest mit 1 bis 6 Kohlenstoffatomen darstellen oder $R_5$ und $R_6$ mit dem Stickstoffatom, an das sie gebunden sind, einen Piperidino- oder Morpholinorest bilden, $R_2$ ein Wasserstoffatom, einen linearen oder verzweigten Alkylrest mit 1 bis 6 Kohlenstoffatomen, einen Arylrest mit 6 bis 12 Kohlenstoffatomen, einen Alkylrest mit 6 bis 12 Kohlenstoffatomen, substituiert durch einen oder mehrere Alkyl- oder Alkoxyreste mit 1 bis 6 Kohlenstoffatomen, durch ein oder mehrere Halogenatome oder durch eine oder mehrere Nitrogruppen, wiedergibt oder $R_2$ einen 2-Thienylrest, einen Aralkylrest mit 7 bis 13 Kohlenstoffatomen, einen Alkoxycarbonylrest mit 2 bis 7 Kohlenstoffatomen oder einen Cycloalkylrest mit 3 bis 6 Kohlenstoffatomen wiedergibt.

2. Derivate der allgemeinen Formel (I) gemäß Anspruch 1 sowie deren Salze, dadurch gekennzeichnet, daß in der Formel (I) R und $R_3$ für ein Wasserstoffatom oder einen Methyl- oder Ethylrest stehen und $R_1$ und $R_2$ die zuvor angegebene Bedeutung besitzen.

3. Derivate der allgemeinen Formel (I) gemäß Anspruch 1 sowie deren Salze, dadurch gekennzeichnet, daß in der Formel (I) R und $R_3$ ein Wasserstoffatom oder einen Methyl- oder Ethylrest bedeutet, $R_1$ die zuvor angegebene Bedeutung besitzt, $R_2$ für ein Wasserstoffatom, einen Alkylrest mit 1 bis 6 Kohlenstoffatomen, einen Phenylrest, einen Phenylrest, substituiert durch eine oder mehrere Methylgruppen, durch ein oder mehrere Halogenatome oder durch einen oder mehrere Nitro- oder Methoxyreste steht oder $R_2$ eine Methoxycarbonylgruppe wiedergibt.

4. Eines der Produkte der Formel (I) gemäß Anspruch 1 mit den folgenden Bezeichnungen :

Ethyl-2-oxo-4-phenyl-2H-pyrimido (2,1-b) benzothiazol-8-acetat,
Ethyl-2-oxo-4-(p-methoxyphenyl)-2H-pyrimido-(2,1-b) benzothiazol-8-acetat,
Ethyl-2-oxo-4-(o-chlorphenyl)-2H-pyrimido(2,1-b) benzothiazol-8-acetat,
Ethyl-α-methyl-2-oxo-4-phenyl-2H-pyrimido(2,1-b)-benzothiazol-8-acetat,
Ethyl-α-methyl-2-oxo-4-(p-chlorphenyl)-2H-pyrimido(2,1-b) benzothiazol-8-acetat,
Ethyl-α-ethyl-2-oxo-4-phenyl-2H-pyrimido(2,1-b) benzothiazol-8-acetat

sowie deren Additionssalze mit Säuren.

5. Verfahren zur Herstellung neuer Pyrimido(2,1-b)-benzothiazolderivate sowie von deren Salzen, dadurch gekennzeichnet, daß man ein Produkt der Formel (II)

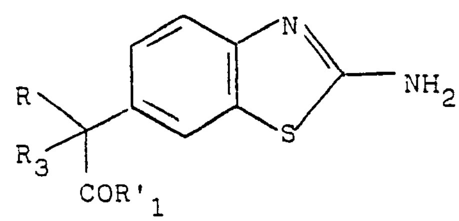
(II)

worin R und $R_3$ wie in Anspruch 1 definiert sind und $R'_1$ für eine Hydroxygruppe oder eine Alkoxygruppe mit 1 bis 6 Kohlenstoffatomen steht, mit einem Produkt der Formel (III)

$$R_2—C\equiv C—CO_2R_4 \qquad (III)$$

worin $R_2$ die angegebene Bedeutung besitzt und $R_4$ einen Alkylrest mit 1 bis 3 Kohlenstoffatomen wiedergibt, umsetzt, um zu einem Produkt der Formel ($I_A$)

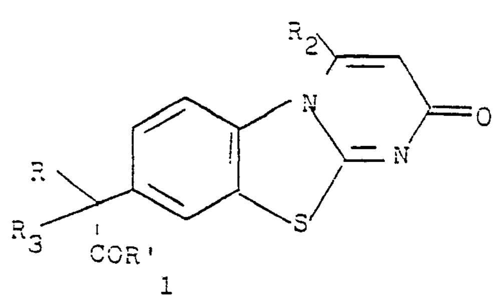

zu gelangen, worin R, $R_3$, $R'_1$ und $R_2$ die angegebene Bedeutung besitzen, und daß man

entweder das so erhaltene Produkt der Formel (I) isoliert und gewünschtenfalls in ein Salz überführt ;

oder ein so erhaltenes Produkt der Formel ($I_A$), in dem $R'_1$ für eine Alkoxygruppe steht, verseift, um zu einem Produkt der Formel ($I_B$)

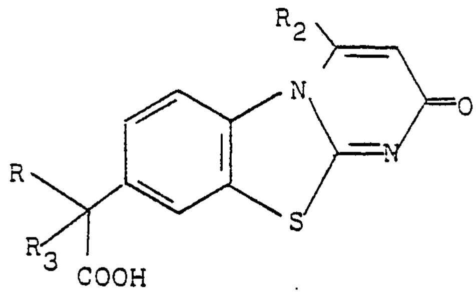

zu gelangen, worin R, $R_3$ und $R_2$ die angegebene Bedeutung besitzen, das man gegebenenfalls isoliert und gewünschtenfalls in ein Salz überführt oder das man durch Umsetzung mit einem Alkohol der Formel (IV)

$$HO—R''_1 \qquad (IV)$$

worin $R''_1$ für eine Alkylgruppe mit 1 bis 6 Kohlenstoffatomen steht, verestert, um ein entsprechendes Produkt der Formel (I) zu erhalten, in dem $R_1$ die Bedeutung von $R''_1$ besitzt, und gewünschtenfalls dieses letztere in ein Salz überführt ;

oder ein Produkt der Formel ($I_A$), in dem $R'_1$ für eine Alkoxygruppe steht, durch Einwirken eines Alkohols der Formel (IV)

$$HO—R''_1 \qquad (IV)$$

worin $R''_1$ die angegebene Bedeutung besitzt, umestert, um zu einem entsprechenden Produkt der Formel (I) zu gelangen, und gewünschtenfalls dieses letztere in ein Salz überführt ;

oder ein Produkt der Formel ($I_A$), worin $R'_1$ für eine Hydroxygruppe steht, mit einem Produkt der Formel

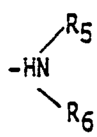

worin $R_5$ und $R_6$ die angegebene Bedeutung besitzen, umsetzt, um zu einem entsprechenden Produkt der Formel (I) zu gelangen, und gewünschtenfalls dieses letztere ein Salz überführt.

6. Verfahren zur Herstellung der neuen Pyrimido(2,1-b)-benzothiazolderivate gemäß Anspruch 5, dadurch gekennzeichnet, daß

a) die Umsetzung des Produktes der Formel (II) mit dem Produkt der Formel (III) in dem Fall, bei dem $R_2$ für ein Wasserstoffatom steht, unter Rückfluß des Reaktionsgemisches in einem Alkohol, wie Ethanol, und in Gegenwart eines Katalysators, wie Palladium, erfolgt ;

b) die Umsetzung des Produkts der Formel (II) mit dem Produkt der Formel (III) in dem Fall, bei dem $R_2$ kein Wasserstoffatom bedeutet, bei einer Temperatur von 100 bis 180 °C in Abwesenheit des Lösungsmittels durchgeführt wird ;

c) die Verseifung des Produkts der Formel ($I_A$) mit einer schwachen Base, wie Kaliumcarbonat, erfolgt ;

d) die Umsetzung des Produkts der Formel ($I_A$) mit dem Produkt der Formel $HNR_5R_6$ in Anwesenheit von 1,1'-Carbodiimidazol erfolgt.

7. Arzneimittel, dadurch gekennzeichnet, daß sie aus den neuen Pyrimido(2,1-b) benzothiazolderivaten der Formel (I) gemäß Anspruch 1 sowie aus deren Additionssalzen mit pharmazeutisch verträglichen Basen und Säuren bestehen.

8. Arzneimittel, dadurch gekennzeichnet, daß sie aus den neuen Derivaten gemäß Anspruch 2, 3 oder 4 sowie aus deren Additionssalzen mit pharmazeutisch verträglichen Basen und Säuren bestehen.

9. Pharmazeutische Zusammensetzungen, dadurch gekennzeichnet, daß sie als Wirkstoff zumindest eines der Arzneimittel gemäß einem der Ansprüche 7 oder 8 enthalten.

10. Produkte der Formel (II)

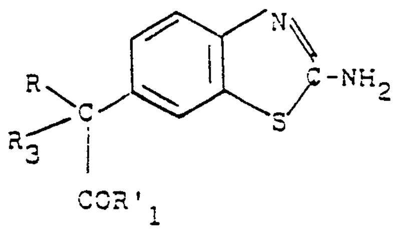

(II)

worin R, $R_3$ und $R'_1$ die in Anspruch 5 angegebene Bedeutung haben, mit der Maßgabe, daß, wenn $R'_1$ eine Hydroxygruppe ist, die Gruppe $R_3RC<$ verschieden ist von $H_3C\text{-}CH<$ oder $H_2C<$, und wenn $R'_1$ eine Thoxygruppe ist, die Gruppe $R_3RC<$ verschieden ist von $H_2C<$.